# EUROPEAN PATENT SPECIFICATION

(11) **EP 0 606 008 B1**
(45) Date of publication and mention of the grant of the patent: **06.03.2002**
(21) Application number: 93310552.0
(22) Date of filing: 24.12.1993
(51) Int. Cl.: C12N 15/56, C07H 21/04, C12N 9/38

(54) **Gene coding for hyperthermostable beta-galactosidase**
Ein für eine hyperthermostabile beta-Galaktosidase kodierendes Gen
Gène codant pour une bêta-galactosidase hyperthermostable

(30) Priority: 08.01.1993 JP 1684693; 06.12.1993 JP 33918893
(43) Date of publication of application: 13.07.1994
(73) Proprietor: TAKARA SHUZO CO. LTD., Fushimi-ku Kyoto 612 (JP)
(72) Inventor: Shimada, Atsushi, Yamtokoriyama-shi, Nara-ken (JP); Odate, Miki, Muko-shi, Kyoto-fu (JP); Koyama, Nobuto, Otsu-shi, Shiga-ken (JP); Hashino, Kimikazu, Takatsuki-shi, Osaka-fu (JP); Asada, Kiyozo, Koga-gun, Shiga-Ken (JP); Kato, Ikunoshin, Uji-shi, Kyoto-fu (JP)
(74) Representative: Wakerley, Helen Rachael

(56) References cited:
- EUR. J. BIOCHEM. vol. 187 , 1990 , SPRINGER VERLAG, BERLIN, BRD; pages 321 - 328 F.M. PISANI ET AL. 'Thermostable beta-galactosidase from the archaebacterium Sulfolobus solfataricus'
- GENE vol. 94 , 1990 , ELSEVIER PUBLISHERS, N.Y., U.S.; pages 89 - 94 M.V. CUBELLIS ET AL. 'Isolation and sequencing of a new beta-galactosidase-encoding archaebacterial gene'
- JOURNAL OF BACTERIOLOGY vol. 174, no. 3 , February 1992 , AM. SOC. MICROBIOL., BALTIMORE, US; pages 873 - 882 M. MORACCI ET AL. 'Expression of the thermostable beta-galactosidase gene from the archaebacterium Sulfolobus solfataricus in Saccharomyces cerevisiae and characterization of a new inducible promoter for heterologous expression'

### SEQUENCE LISTING

INFORMATION FOR SEQ ID NO:1:
   (i) SEQUENCE CHARACTERISTICS:
      (A) LENGTH: 510 amino acids
      (B) TYPE: amino acid
      (C) STRANDEDNESS: single
      (D) TOPOLOGY: linear
   (ii) MOLECULE TYPE:peptide
   (iii) HYPOTHETICAL:
   (iv) ANTI-SENSE:
   (v) FRAGMENT TYPE:
   (vi) ORIGINAL SOURCE:
      (A) ORGANISM: *Pyrococcus furiosus*
      (B) STRAIN:
      (C) INDIVIDUAL ISOLATE:
      (D) DEVELOPMENTAL STAGE:
      (E) HAPLOTYPE:
      (F) TISSUE TYPE:
      (G) CELL TYPE:
      (H) CELL LINE:
      (I) ORGANELLE:
   (vii) IMMEDIATE SOURCE:
      (A) LIBRARY:
      (B) CLONE:
   (viii)POSITION IN GENOME:
      (A) CHROMOSOME/SEGMENT:
      (B) MAP POSITION:
      (C) UNITS:
   (ix) FEATURE:
      (A) NAME/KEY:
      (B) LOCATION:
      (C) IDENTIFICATION METHOD:
      (D) OTHER INFORMATION:
   (x) PUBLICATION INFORMATION:
      (A) AUTHORS:
      (B) TITLE:
      (C) JOURNAL:
      (D) VOLUME:
      (E) ISSUE:
      (F) PAGES:
      (G) DATE:
      (H) DOCUMENT NUMBER:
      (I) FILING DATE:
      (J) PUBLICATION DATE:
      (K) RELEVANT RESIDUES:
   (xi) SEQUENCE DESCRIPTION: SEQ ID NO:1:
   Met Phe Pro Glu Lys Phe Leu Trp Gly Val Ala Gln Ser Gly Phe
    1               5                  10                  15
   Gln Phe Glu Met Gly Asp Lys Leu Arg Arg Asn Ile Asp Thr Asn
                   20                  25                  30
   Thr Asp Trp Trp His Trp Val Arg Asp Lys Thr Asn Ile Glu Lys
                   35                  40                  45
   Gly Leu Val Ser Gly Asp Leu Pro Glu Glu Gly Ile Asn Asn Tyr
                   50                  55                  60
   Glu Leu Tyr Glu Lys Asp His Glu Ile Ala Arg Lys Leu Gly Leu
                   65                  70                  75
   Asn Ala Tyr Arg Ile Gly Ile Glu Trp Ser Arg Ile Phe Pro Trp
                   80                  85                  90
   Pro Thr Thr Phe Ile Asp Val Asp Tyr Ser Tyr Asn Glu Ser Tyr
                   95                  100                 105
   Asn Leu Ile Glu Asp Val Lys Ile Thr Lys Asp Thr Leu Glu Glu
                   110                 115                 120
   Leu Asp Glu Ile Ala Asn Lys Arg Glu Val Ala Tyr Tyr Arg Ser
                   125                 130                 135
   Val Ile Asn Ser Leu Arg Ser Lys Gly Phe Lys Val Ile Val Asn
                   140                 145                 150
   Leu Asn His Phe Thr Leu Pro Tyr Trp Leu His Asp Pro Ile Glu
                   155                 160                 165
   Ala Arg Glu Arg Ala Leu Thr Asn Lys Arg Asn Gly Trp Val Asn
                   170                 175                 180
   Pro Arg Thr Val Ile Glu Phe Ala Lys Tyr Ala Ala Tyr Ile Ala
                   185                 190                 195
   Tyr Lys Phe Gly Asp Ile Val Asp Met Trp Ser Thr Phe Asn Glu
                   200                 205                 210
   Pro Met Val Val Val Glu Leu Gly Tyr Leu Ala Pro Tyr Ser Gly
                   215                 220                 225
   Phe Pro Pro Gly Val Leu Asn Pro Glu Ala Ala Lys Leu Ala Ile
                   230                 235                 240
   Leu His Met Ile Asn Ala His Ala Leu Ala Tyr Arg Gln Ile Lys
                   245                 250                 255
   Lys Phe Asp Thr Glu Lys Ala Asp Lys Asp Ser Lys Glu Pro Ala
                   260                 265                 270
   Glu Val Gly Ile Ile Tyr Asn Asn Ile Gly Val Ala Tyr Pro Lys
                   275                 280                 285
   Asp Pro Asn Asp Ser Lys Asp Val Lys Ala Ala Glu Asn Asp Asn
                   290                 295                 300
   Phe Phe His Ser Gly Leu Phe Phe Glu Ala Ile His Lys Gly Lys
                   305                 310                 315
   Leu Asn Ile Glu Phe Asp Gly Glu Thr Phe Ile Asp Ala Pro Tyr
                   320                 325                 330
   Leu Lys Gly Asn Asp Trp Ile Gly Val Asn Tyr Tyr Thr Arg Glu
                   335                 340                 345
   Val Val Thr Tyr Gln Glu Pro Met Phe Pro Ser Ile Pro Leu Ile
                   350                 355                 360
   Thr Phe Lys Gly Val Gln Gly Tyr Gly Tyr Ala Cys Arg Pro Gly
                   365                 370                 375
   Thr Leu Ser Lys Asp Asp Arg Pro Val Ser Asp Ile Gly Trp Glu
                   380                 385                 390
   Leu Tyr Pro Glu Gly Met Tyr Asp Ser Ile Val Glu Ala His Lys
                   395                 400                 405
   Tyr Gly Val Pro Val Tyr Val Thr Glu Asn Gly Ile Ala Asp Ser
                           410                     415                     420
   Lys Asp Ile Leu Arg Pro Tyr Tyr Ile Ala Ser His Ile Lys Met
                           425                     430                     435
   Ile Glu Lys Ala Phe Glu Asp Gly Tyr Glu Val Lys Gly Tyr Phe
                           440                     445                     450
   His Trp Ala Leu Thr Asp Asn Phe Glu Trp Ala Leu Gly Phe Arg
                           455                     460                     465
   Met Arg Phe Gly Leu Tyr Glu Val Asn Leu Ile Thr Lys Glu Arg
                           470                     475                     480
   Ile Pro Arg Glu Lys Ser Val Ser Ile Phe Arg Glu Ile Val Ala
                           485                     490                     495
   Asn Asn Gly Val Thr Lys Lys Ile Glu Glu Glu Leu Leu Arg Gly
                           500                     505                     510
(2) INFORMATION FOR SEQ ID NO:2:
   (i) SEQUENCE CHARACTERISTICS:
      (A) LENGTH: 1533 base pairs
      (B) TYPE: nucleic acid
      (C) STRANDEDNESS: double
      (D) TOPOLOGY: linear
   (ii) MOLECULE TYPE: genomic DNA
   (iii) HYPOTHETICAL: NO
   (iv) ANTI-SENSE: NO
   (v) FRAGMENT TYPE:
   (vi) ORIGINAL SOURCE:
      (A) ORGANISM:*Pyrococcus furiosus*
      (B) STRAIN:
      (C) INDIVIDUAL ISOLATE:
      (D) DEVELOPMENTAL STAGE:
      (E) HAPLOTYPE:
      (F) TISSUE TYPE:
      (G) CELL TYPE:
      (H) CELL LINE:
      (I) ORGANELLE:
   (vii) IMMEDIATE SOURCE:
      (A) LIBRARY:
      (B) CLONE:
   (viii)POSITION IN GENOME:
      (A) CHROMOSOME/SEGMENT:
      (B) MAP POSITION:
      (C) UNITS:
   (ix) FEATURE:
      (A) NAME/KEY:
      (B) LOCATION:
      (C) IDENTIFICATION METHOD:
      (D) OTHER INFORMATION:
   (x) PUBLICATION INFORMATION:
      (A) AUTHORS:
      (B) TITLE:
      (C) JOURNAL:
      (D) VOLUME:
      (E) ISSUE:
      (F) PAGES:
      (G) DATE:
      (H) DOCUMENT NUMBER:
      (I) FILING DATE:
      (J) PUBLICATION DATE:
      (K) RELEVANT RESIDUES:
   (xi) SEQUENCE DESCRIPTION: SEQ ID NO:2:
   ATGTTCCCTG AAAAGTTCCT TTGGGGTGTG GCACAATCGG GTTTTCAGTT TGAAATGGGG   60
   GATAAACTCA GGAGGAATAT TGACACTAAC ACTGATTGGT GGCACTGGGT AAGGGATAAG  120
   ACAAATATAG AGAAAGGCCT CGTTAGTGGA GATCTTCCCG AGGAGGGGAT TAACAATTAC  180
   GAGCTTTATG AGAAGGACCA TGAGATTGCA AGAAAGCTGG GTCTTAATGC TTACAGAATA  240
   GGCATAGAGT GGAGCAGAAT ATTCCCATGG CCAACGACAT TTATTGATGT TGATTATAGC  300
   TATAATGAAT CATATAACCT TATAGAAGAT GTAAAGATCA CCAAGGACAC TTTGGAGGAG  360
   TTAGATGAGA TCGCCAACAA GAGGGAGGTG GCCTACTATA GGTCAGTCAT AAACAGCCTG  420
   AGGAGCAAGG GGTTTAAGGT TATAGTTAAT CTAAATCACT TCACCCTTCC ATATTGGTTG  480
   CATGATCCCA TTGAGGCTAG GGAGAGGGCG TTAACTAATA AGAGGAACGG CTGGGTTAAC  540
   CCAAGAACAG TTATAGAGTT TGCAAAGTAT GCCGCTTACA TAGCCTATAA GTTTGGAGAT  600
   ATAGTGGATA TGTGGAGCAC GTTTAATGAG CCTATGGTGG TTGTTGAGCT TGGCTACCTA  660
   GCCCCCTACT CTGGCTTCCC TCCAGGGGTT CTAAATCCAG AGGCCGCAAA GCTGGCGATA  720
   CTTCACATGA TAAATGCACA TGCTTTAGCT TATAGGCAGA TAAAGAAGTT TGACACTGAG  780
   AAAGCTGATA AGGATTCTAA AGAGCCTGCA GAAGTTGGTA TAATTTACAA CAACATTGGA  840
   GTTGCTTATC CCAAGGATCC GAACGATTCC AAGGATGTTA AGGCAGCAGA AAACGACAAC  900
   TTCTTCCACT CAGGGCTGTT CTTCGAGGCC ATACACAAAG GAAAACTTAA TATAGAGTTT  960
   GACGGTGAAA CGTTTATAGA TGCCCCCTAT CTAAAGGGCA ATGACTGGAT AGGGGTTAAT 1020
   TACTACACAA GGGAAGTAGT TACGTATCAG GAACCAATGT TTCCTTCAAT CCCGCTGATC 1080
   ACCTTTAAGG GAGTTCAAGG ATATGGCTAT GCCTGCAGAC CTGGAACTCT GTCAAAGGAT 1140
   GACAGACCCG TCAGCGACAT AGGATGGGAA CTCTATCCAG AGGGGATGTA CGATTCAATA 1200
   GTTGAAGCTC ACAAGTACGG CGTTCCAGTT TACGTGACGG AGAACGGAAT AGCGGATTCA 1260
   AAGGACATCC TAAGACCTTA CTACATAGCG AGCCACATAA AGATGATAGA GAAGGCCTTT 1320
   GAGGATGGGT ATGAAGTTAA GGGCTACTTC CACTGGGCAT TAACTGACAA CTTCGAGTGG 1380
   GCTCTCGGGT TTAGAATGCG CTTTGGCCTC TACGAAGTCA ACCTAATTAC AAAGGAGAGA 1440
   ATTCCCAGGG AGAAGAGCGT GTCGATATTC AGAGAGATAG TAGCCAATAA TGGTGTTACG 1500
   AAAAAGATTG AAGAGGAATT GCTGAGGGGA TGA                             1533

## Description

This invention relates to a gene coding for a hyperthermostable β-galactosidase and a genetic engineering process for producing the enzyme which is useful in the fields of, for example, food industry and saccharide engineering.

β-Galactosidase, which is an enzyme capable of decomposing β-galactoside, has been found out in animals, plants and microorganisms. It is known that this enzyme occurs particularly in bacteria such as *Escherichia coli*, *Streptococcus lactis, Bacillus subtillis, Streptococcus thermoshilus* and *Sulfolobus solfataricus*. This β-galactosidase is applied to the production of low-lactose milk by taking advantage of its ability to hydrolyze lactose into galactose and glucose. It is also applied to the production of galactose or glucose from lactose contained in milk serum which is formed in a large amount in the process of producing cheese.

To apply β-galactosidase to food processing, therefore, it has been demanded to develop an enzyme which can withstand the use at a high temperature from the viewpoint of preventing contamination with microorganisms during the processing and another viewpoint of elevating the solubility of lactose which serves as a substrate. For example, a β-galactosidase originating in *sulfolobus solfataricus* [see European Journal of Biochemistry, 187, 321 - 328 (1990)] is a thermophilic enzyme having an activity at a temperature of 90°C. However, its activity falls to about 50% after treating at 85°C for 180 minutes.

As mentioned above, a thermophilic and thermostable enzyme has been demanded in food processing at a high temperature.

In order to solve the problem as mentioned above, the present invention aims at isolating a gene coding for β-galactosidase having an improved thermophilicity and an excellent thermostability and providing an process for producing a hyperthermostable β-galactosidase with the use of the gene.

The first aspect of the present invention provides an isolated DNA having:
(a) the DNA sequence of SEQ ID No. 2, or a fragment thereof;
(b) a DNA sequence encoding the amino acid sequence of SEQ ID No. 1 or a fragment thereof;
(c) a DNA sequence encoding the amino acid sequence resulting from deletion, addition, insertion, or substitution, of one or more amino acids in the amino acid sequence of SEQ ID No. 1; or
(d) a DNA sequence capable of hybridizing of any one of (a) to (c) under stringent conditions;
wherein said DNA sequence or fragment thereof encode a polypeptide possessing hyperthermostable β-galactosidase activity of about 100% after having been treated at 90°C for 120 minutes.

The present invention further provides a process for producing a hyperthermostable β-galactosidase which having the activity of about 100% after having been treated at 90°C for 120 minutes, which comprises:
(a) constructing a plasmid in which an isolated DNA encoding a hyperthermophilic β-galactosidase according to the first aspect of the invention is inserted;
(b) transforming an appropriate microorganism with said plasmid; and
(c) cultivating the transformed microorganism and recovering the hyperthermostable β-galactosidase from the culture.

The hyperthermostable p-galactosidase gene which includes an isolated DNA encoding a hyperthermophilic β-galactosidase in this invention can be screened and obtained by the expression cloning method using cosmid vectors. Expression cloning is a method which can be used for cloning of the gene coding some enzymes without any information about the primary structure of the target enzyme. For example, a pullulanase gene of Pyrococcus furiosus (WO 92/02614) is cloned using the expression cloning method. However, the method cannot be applied to cloning of any type of enzyme because in case the plasmid vector is used for the method, a very suitable restriction enzyme is needed; It must cleave the target gene into small size enough to be inserted in a plasmid vector and neither cleave the target gene at inside. Furthermore, the method is complicated because it needs a number of clones.

Subsequently, the present inventors have attempted to isolate the β-galactosidase gene by screening β-galactosidase activities in a cosmid library constructed with *Pyrococcus furiosus* genomic DNA and the cosmid vectors in which larger DNA fragments (35-50kbp) can be inserted than in plasmid vectors. By using cosmid vectors, dangers for cleaving the target gene coding the enzyme by a restriction enzyme at inside decrease and the numbers of clones necessary to test can be reduced. On the contrary, dangers not to detect the enzyme activity cause because of low expression of the enzyme because the cosmid vectors has less copy numbers in host organisms than the plasmid vectors.

The present inventors sited in extreme high thermostability of the target enzyme and combined a process of cultivating the transformants in the cosmid library individually with a process of preparing the lysates which contain only the thermostable proteins. The group of these lysates is named as "cosmid protein library". By using the library for detection of the enzyme activity, detection sensitivity increases than using colonies of the transformants and bad influences such as background by proteins from hosts or inhibition of enzyme activity can be deleted. The present inventors isolated several cosmid clones which show β-galactosidase activity by screening the cosmid protein library constructed with *Pyrococcus furiosus* genomic DNA. Furthermore, the present inventors isolated the gene coding a hyperthermostable β-galactosidase from the DNA fragments inserted in the clones isolated above by making full use of various genetic engineering techniques, and determined the DNA sequence of the gene. And more, the present inventors succeeded in the expression of the hyperthermostable β-galactosidase with the use of the gene, thus completing the present invention.

By the way, the expression cloning method using cosmid vectors which is described here cannot be always applied to any thermostable enzyme. The result is determined by the property of the target gene. For the example, the present inventors attempted to isolate the gene coding a protease of *Pyrococcus* furiosus (Appl. Env. Microbiol.56,1992-1998(1990)), but they *didrit* reach to the isolation of the gene.

Now, the present invention will be described in greater detail.

The microorganism to be used in the present invention is not particularly restricted, so long as it can produce a hyperthermostable β-galactosidase gene. For example, strains belonging to the genus *Pyrococcus*, i.e., hyperthermostable bacteria, such as *Pyrococcus furiosus* DMS 3638 and *Pyrococcus woesei* DMS 3773 are usable therefor. These strains are both available from Deutsche Sammlung von Mikroorganismen und Zellkulturen GmbH.

For example, a cosmid library of *Pyrococcus* furiosus gene can be prepared in the following manner. First, the genome gene of *Pyrococcus furiosus* 3638 is partially digested by using an appropriate restriction enzyme, for example, Sau 3AI (mfd. by Takara Shuzo Co., Ltd.). After fractionating according to the size of 35 to 50 kbp, each DNA fragment thus obtained is ligated with an appropriate cosmid vector, for example, Triple Helix Cosmid Vector (mfd. by Stratagene). The *Pyrococcus furiosus* genome DNA fragments are first packaged in λ-phage particles by the *in vitro* packaging method and then an appropriate *Escherichia coli* strain, for example, *E. coli* DH5αMCR (mfd. by BRL) is transformed with the obtained phage solution to thereby give the aimed cosmid library. Then cosmid DNAs are prepared from several colonies of the transformant and the insertion of the genome DNA fragments of 35 to 50 kbp into the transformant is thus confirmed. In general, 300 to 700 colonies may be incubated.

After the completion of the incubation of each colony, the incubated cells are collected. The cells are processed to the cosmid protein libraries by treating at 100°C for 10 minutes, sonicating, and treating at 100°C for 10 minutes once more. Then the β-galactosidase activity in the lysates obtained is determined, whereby colonies expressing a hyperthermostable β-galactosidase which remains stable after treatment above described can be screened. The β-galactosidase activity is determined by, for example, using o-nitrophenyl-β-D-galactopyranoside or lactose as a substrate at a reaction temperature of, for example, 95°C. Next, the fragment inserted into the cosmid DNA of transformant showing the activity is analysed.

Inserted fragments in the cosmid DNAs of seven active transformants, among 500 transformants prepared by the present inventors, contain an EcoRI fragment of about 2.2 kbp in common. Thus this common DNA fragment is prepared to examine its properties. For example, a cosmid DNA expressing the activity is completely digested with a restriction enzyme EcoRI (mfd. by Takara Shuzo Co., Ltd.) and electrophoresed on a gel to thereby purify the target EcoRI DNA fragment of about 2.2 kbp. Then the obtained fragment is inserted into the EcoRI site of an appropriate vector, for example, pUC19 (mfd. by Takara Shuzo Co., Ltd.). Thus a plasmid holding the common DNA fragment, which has been named pTGE-101 by the present inventors, can be prepared. By this plasmid pTGE-101, an appropriate *Escherichia coli* strain such as *E. coli* JM109 is transformed. Thus a transformant named *Escherichia coli* JM109/pTGE-101 by the present inventors can be obtained. After incubating this *E. coli* transformant, the cells are collected and the β-galactosidase activity of the protein expressed in the cells is determined. The activity can be determined in the following manner. Namely, the cells and disrupted cells, which have been either thermally treated at 100°C for 10 minutes, twice or untreated, are used and the above-mentioned o-nitrophenyl-β-D-galactopyranoside is employed as a substrate to effect a reaction at 70°C or 95°C, whereby the thermostability of the expressed protein can be examined. As Table 1 shows, a gene coding for a thermostable β-galactosidase, which is inactivated when treated at 100°C for 10 minutes, twice but exhibits its activity at a reaction temperature of 70°C, has been inserted into pTGE-101.

**Table 1**

| Thermal treatment (100°C, 10minutes, twice) | Reaction temperature | |
|---|---|---|
| | 70°C | 95°C |
| Treated | - | - |
| Haven't been treated | + | - |

In the above Table, symbol + means the presence of β-galactosidase activity, while symbol - means the absence thereof.

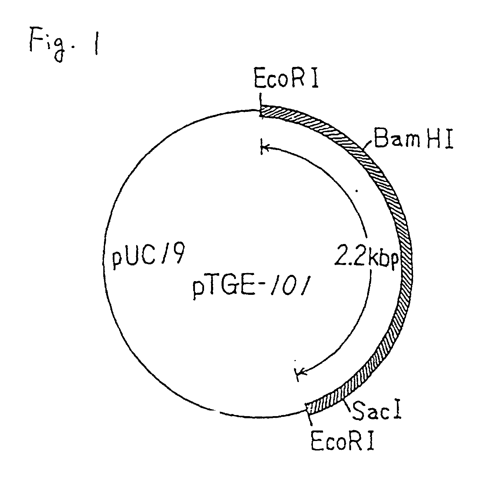

Fig. 1 shows a restriction enzyme map of the plasmid pTGE-101 wherein a thick solid line represents the EcoRI DNA fragment of about 2.2 kbp inserted into the plasmid pUC19. This fragment codes for the thermostable β-galactosidase. This fragment further codes for a part of the target hyperthermostable β-galactosidase.

The above-mentioned EcoRI DNA fragment of about 2.2 kbp is labeled. By using the labeled DNA fragment thus obtained as a probe, DNA fragments, which have been obtained by completely digesting cosmids having a hyperthermostable β-galactosidase gene with a restriction enzyme Hind III (mfd. by Takara Shuzo Co., Ltd.) and separating by agarose gel electrophoresis, are subjected to the southern hybridization. Thus a Hind *III DNA* fragment (about 3.5 kbp) hybridized with the probe can be identified. Then this fragment is extracted from the agarose gel and inserted into the Hind III site of an appropriate vector, for example, pUC19. Thus a plasmid named pTGH-301 by the present inventors can be prepared. By transforming *E. coli* JM109 by this plasmid, a transformant named *Escherichia coli* JM109/pTGH-301 by the present inventors can be obtained. This transformant is incubated and, after the completion of the incubation, the cells are collected. The β-galactosidase expressed in these cells remains stable after treating at 100°C for 10 minutes twice. Thus the target hyperthermostable β-galactosidase has been expressed therein.

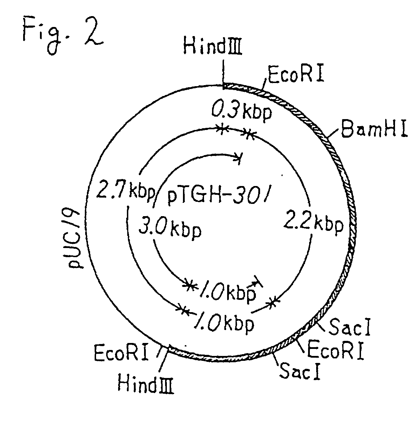

Fig. 2 shows a restriction enzyme map of the plasmid pTGH-301 wherein a thick solid line represents the Hind III DNA fragment of about 3.5 kbp inserted into the plasmid pUC19. This fragment contains a hyperthermostable β-galactosidase gene. This fragment also contains the EcoRI DNA fragment of about 2.2 kbp shown in Fig. 1.

A part of the DNA sequence coding the hyperthermostable β-galactosidase which is contained in plasmid pTGH-301 is shown in SEQ ID No.2 of the sequence listing. SEQ ID No.1 of the sequence listing shows the amino acid sequence presumed from the DNA sequence shown in SEQ ID No.1. Namely, SEQ ID NO. 1 of the sequence listing is the amino acid sequence of the hyperthermostable β-galactosidase which is produced with the use of an isolated DNA encoding a hyperthermostable β-galactosidase of the present invention. The isolated DNA encoding a hyperthermostable β-galactosidase of the present invention is also provided as an isolated DNA which has a DNA sequence encoding the amino acid sequence shown in SEQ ID NO.1 of the sequence listing consisting of any combinations of genetic codes selected from degenerated codons.

Namely, the DNA sequence shown in SEQ ID No.2 of the sequence listing is an example of the hyperthermostable β-galactosidase gene given in present invention.

And more, N-terminal amino acid sequence of the purified sample of the hyperthermostable β-galactosidase produced by *Escherichia coli* JM109 /pTGH301 agreed with the N-terminal amino acid sequence of the amino acid sequence shown in SEQ ID NO.1 of the sequence listing.

From this plasmid pTGH-301, a Hind III-EcoRI DNA fragment (about 1.0 kbp) free from the hyperthermostable β-galactosidase gene can be eliminated in the following manner.

Namely, from 3 types of DNA fragments obtained by digesting the plasmid pTGH-301 with EcoRI, only the one of about 1.0 kbp is eliminated and ligation is effected, followed by transduction into *E. coli* JM109. Then the hyperthermostable β-galactosidase activities of the obtained colonies are determined and a plasmid is prepared from a colony showing an activity.

This plasmid is named pTGEH-401, while *E. coli* JM109 transformed by this plasmid is named *Escherichia coli* JM109/pTGEH-401, and deposited at the National Institute of Bioscience and Human-Technology Agency of Industrial Science and Technology, Japan, under the accession number of FERM BP-4468.

Fig. 3 shows a restriction enzyme map of the plasmid pTGEH-401, wherein a thick solid line represents the DNA fragment inserted into the plasmid pUC19.

Fig. 4 shows a restriction enzyme map of a *DNA* fragment originating in *Pyrococcus furiosus* which has been inserted into the plasmid pTGEH-401. Namely, Fig. 4 shows the restriction enzyme map of one example of the hyperthermostable β-galactosidase gene obtained according to the present invention.

When a transformant constructed by transducing a recombinant plasmid containing a hyperthermostable β-galactosidase gene such as *Escherichia coli* JM109/pTGH-301 or *Escherichia coli* JM109/pTGEH-401 is incubated, the hyperthermostable β-galactosidase is accumulated in the culture. The hyperthermostable β-galactosidase can be purified from this culture by, for example, harvesting the cells, disrupting the cells by ultrasonication and treating the supernatant obtained by centrifuging by a combination of purification procedures such as gel filtration chromatography, ion exchange chromatography and hydrophobic chromatography.

When the hyperthermostable β-galactosidase is to be purified in the present invention, in particular, it is advantageous to thermally treat the cells either before or after the ultrasonication, since the contaminating proteins are denatured thereby and thus the purification can be easily carried out.

The hyperthermostable β-galactosidase obtained by expressing a gene of the present invention, for example, a gene integrated in the plasmid pTGH-301 has the following physicochemical properties.

### (1) Action and substrate specificity:

It has an action of hydrolyzing lactose into galactose and glucose. Further, it has an action of hydrolyzing o-nitrophenyl-β-D-galactopyranoside into o-nitrophenol and galactose.

### (2) Method for determining enzymatic activity:

### [(2)-a]

In the determination of enzymatic activity, the o-nitro-phenyl-β-D-galactopyranoside-hydrolyzing activity of an enzyme can be determined by spectroscopically monitoring o-nitrophenol formed via the hydrolysis. Namely, 5 µl of the enzyme solution of the present invention is added to 199 µl of a 100 mM phosphate buffer solution (pH 7.3) containing 112 mM of 2-mercaptoethanol and 1 mM of magnesium chloride. Then 1 µl of a dimethyl sulfoxide solution containing 0.4 M of o-nitrophenyl-β-D-galactopyranoside is added thereto. After effecting a reaction at 95°C for 30 minutes, the reaction is ceased by adding 100 µl of 0.1 M sodium carbonate and the absorbance of the reaction mixture at 410 nm is measured to thereby determine the amount of the o-nitrophenol thus formed. One unit of the hyperthermostable β-galactosidase obtained in accordance with the present invention is expressed in an amount of the enzyme whereby the absorbance at 410 nm can be increased by 1.0 at 95°C within 1 minute. The enzyme obtained in the present invention has an activity of decomposing o-nitrophenyl-β-D-galactopyranoside at pH 7.3 at 95°C, as measured above.

### [(2)-b]

The o-nitrophenyl-β-D-galactopyranoside-hydrolyzing activity of the β-galactosidase also can be determined by the method shown as follows; The enzyme reaction was started by adding 15 µl of a dimethyl sulfoxide solution containing 1 M of o-nitrophenyl-β-D-galactopyranoside into 1485 µl of McIlvaine's buffer solution containing the enzyme which is in a quartz cuvette for spectrometer to give the final concentration of o-nierophenyl-β-D-galactopyranoside to 10mM. Reaction was detected by monitoring change of absorbance at 410nm versus time on spectrophotometer. Based on the change of absorbance at 410nm per minute, o-nitrophenol released per minute was calculated by using absorbance coefficient determined previously. One unit of enzyme activity was defined as that amount required to catalyze the release of 1 µmol o-nitrophenol per minute. Protein determination of the enzyme protein was performed by the method of Lowly's.

### [2-c]

Further, the enzymatic activity of the enzyme obtained in the present invention can be determined also by measuring the activity of decomposing lactose. The activity of decomposing lactose is measured by determining the amount of glucose formed by the decomposition of the substrate, as will be described hereinbelow. 5 µl of the enzyme solution is added to 70 µl of a 100 mM glycine sodium buffer solution (pH 7.5) containing 4 mM of magnesium sulfate. Next, 25 µl of a 2% lactose solution is added thereto and the mixture is reacted at 95°C for 30 minutes. After ceasing the reaction by cooling with ice, the amount of the liberated glucose is determined by using a glucose measurement kit Glucose B-Test Wako (mfd. by Wako Pure Chemical Industries, Ltd.). The enzyme obtained in the present invention has an activity of decomposing lactose at pH 7.5 at 95°C.

### (3) Thermostability:

By using 32 mU of the enzyme obtained in the present invention, the thermostability of the enzyme is measured. The measurement is effected by determining the o-nitrophenyl-β-D-galactopyranoside-hydrolyzing activity of the enzyme in accordance with the method described in the above[(2)-a] except that the phosphate buffer solution is replaced by McIlvaine's buffer solution (pH 6.6) containing 112 mM of 2-mercapto-ethanol and 1 mM of MgCl₂. After treating the enzyme in 204 µl of the above-mentioned buffer solution at 90°C for various periods of time, 1 µl of a dimethyl sulfoxide solution containing 0.4 M of o-nitrophenyl-β-D-galactopyranoside is added, followed by a reaction at 95°C for 30 minutes. Then the reaction is ceased by adding 100 µl of 0.1 M sodium carbonate and the absorbance at 410 nm is measured to thereby determine the amount of the o-nitrophenol thus formed.

The enzyme obtained in the present invention exhibits a hyperthermostability and suffers from no decrease in the enzymatic activity even after treating at 90°C for 120 minutes.

The thermostability of the β-galactosidase was determined in accordance with the described in [2-b]. 1 ml of McIlvaine's buffer solution containing the enzyme (pH 5.0) was incubated at 90°C and aliquots were withdrawn at appointed times. The enzyme reaction was started by adding 10 µl of the aliquot to 1490 µl of McIlvaine's buffer solution (pH 5.0) containing 10 mM of o-nitroghenyl-β-D-galactopyranoside which had been incubated at 90°C in the cuvette.

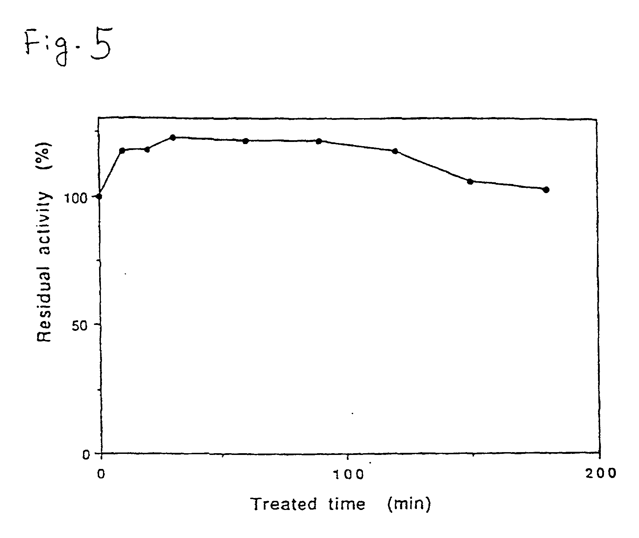

Reaction was detected by monitoring of absorbance at 410nm versus time on spectrophotometer. Based on the change of absorbance at 410nm per minute, o-nitrophenol released per a minute was calculated by using absorbance coefficient determined previously. One unit of enzyme activity was defined as that amount required to catalyze the release of 1 µmol o-nitrophenol per minute. As Fig. 5 shows, about 100% of activity of the β-galactosidase at ratio(%) was retained even after treating at 90°C for 150 min. Fig.5 is a graph showing the thermostability of the β-galactosidase wherein the ordinate refers to the residual activity ratio (%), while the abscissa refers to treating time (minute) at 90°C.

### (4) Optimum pH value;

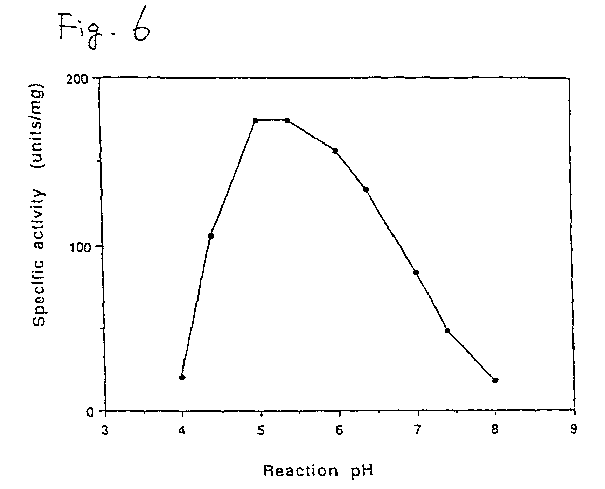

The optimum pH value of the β-galactosidase were measured in accordance with the method described in the above [(2)-b]. 1490 µl of McIlvaine's buffer solution which was determined pH at appointed value (pH 4-8) and containing 10 mM of o-nitrophenyl-β-D-galactopyranoside was incubated at 90°C in the cuvette and the enzyme reaction was started by adding 10 µl of McIlvaine's buffer solution (pH 5.0) containing the enzyme into the cuvette. Reaction was detected by monitoring change of absorbance at 410nm versus time on spectrophotometer. Change of absorbance at 410nm per minute was determined. Based on the change of absorbance at 410nm per minute, o-nitrophenol released per minute was calculated by using absorbance coefficient determined at each pH condition. One unit of enzyme activity was defined as that amount required to catalyze the release of 1 µmol o-nitrophenol in a minute. As shown in Fig. 6, the β-galactosidase shows its maximum activity at a pH range of from 4.5 to 6.5. Fig. 6 is a graph showing the optimum pH value of the β-galactosidase wherein the ordinate refers to the specific activity (units/mg protein), while the abscissa refers to treating pH.

### (5) Optimum temperature:

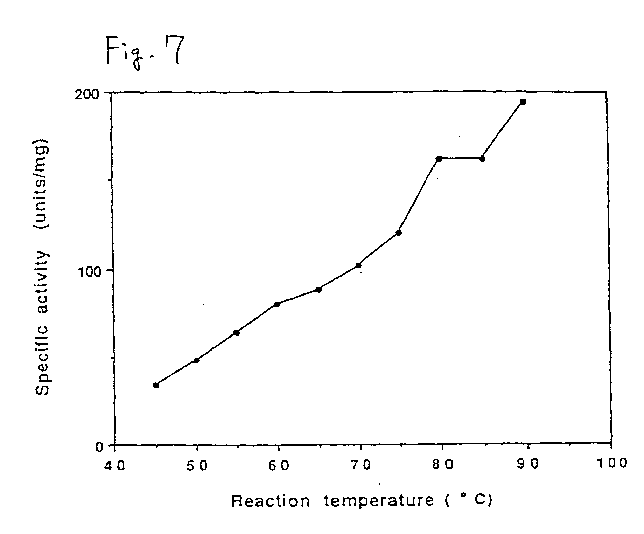

The optimum temperatures of the β-galactosidase was measured in accordance with the described in [2-b]. 1485 µl of McIlvaine's buffer solution (pH 5.0) containing the β-galactosidase was incubated at appointed temperature (45°C -90°C) in the cuvette and the enzyme reaction was started by adding 15 µl of a dimethyl sulfoxide solution containing 1 M of o-nitrophenyl-β-D-galactopyranoside to give the final concentration of o-nitrophenyl-β-D-galactopyranoside to 10mM. Reaction was detected by monitoring change of absorbance at 410nm versus time on spectrophotometer. Change of absorbance at 410nm per minute was determined. Based on the change of absorbance at 410nm per minute, o-nitrophenol released per minute was calculated by using absorbance coefficient determined at each temperature. One unit of enzyme activity was defined as that amount required to catalyze the release of 1 µmol o-nitrophenol per minute. As Fig. 7 shows, the β-galactosidase shows its maximum activity above 90°C. Fig.7 is a graph showing the optimum temperature of the β-galactosidase wherein the ordinate refers to the specific activity (units/mg protein), while the abscissa refers to treating temperature (°C).

### (6) pH stability:

The pH stability of the β-galactosidase are determined in accordance with the described in [2-b]. 1485 µl of McIlvaine's buffer solution containing 20 units/ml of the β-galactosidase was incubated at appointed pH (pH 3-8) for 10 minutes at 90°C. To start reaction, 10µl of aliquot of the enzyme solution was added to 1490µl of McIlvaine's buffer solution (pH 5.0) which contained 10mM of o-nitrophenyl-β-D-galactopyranoside and preincubated at 90°C.

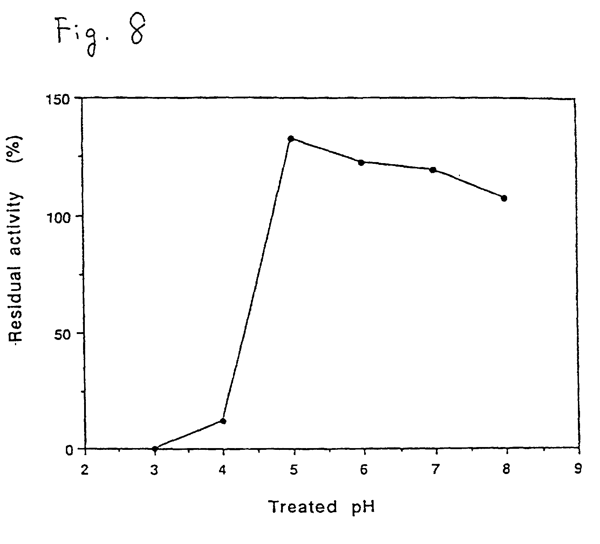

Reaction was detected by monitoring change of absorbance at 410nm versus time on spectrophotometer. Change of absorbance at 410nm in a minute was determined. Based on the change of absorbance at 410nm per minute, o-nitrophenol released per minute was calculated by using absorbance coefficient determined previously. One unit of enzyme activity was defined as that amount required to catalyze the release of 1 µmol o-nitrophenol in a minute. As Fig shows, the β-galactosidase sustains its activity even after treating within a pH range of from 4.5 to 8.0 at 90°C for 10 minutes. Fig. 8 is a graph showing the optimum pH value of the β-galactosidase wherein the ordinate refers to the residual activity ratio(%), while the abscissa refers to treating pH.

### (7) Substrate specificity:

Substrate specificity of the β-galactosidase is able to be determined by using p-nitrophenol-derivatives as shown in Table 2.
The method is shown as follows;
1485 µl of 150 mM sodium citrate buffer solution (pH 5.0) containing the β-galactosidase is added to a quartz cuvette for spectrometer. 15µl of 0.1M substrate solution shown in Table 2 is added to the enzyme solution and mixed. Immediately, reaction was detected by monitoring change of absorbance at 410nm versus time on spectrophotometer. As a blank test, 1485 µl of 150 mM sodium citrate buffer (pH 5.0) not containing enzyme was used, and determination described above was performed. On the test, reaction was performed at 90°C. One unit of enzyme activity was defined as that amount required to catalyze the release of 1 µmol p-nitrophenol per minute.

According to the method described above, Hydrolytic activity towards p-nitrophenyl-β-D-glucopyranoside(GlcpβNp), p-nitrophenyl-β-D-galactopyranoside(GalpβNp), p-nitrophenyl-β-D-mannopyranoside(ManpβNp), p-nitrophenyl-β-D-xylopyranoside(XylpβNp), p-nitrophenyl-β-D-fucopyranoside(FucpβNp), p-nitrophenyl-α-D-galactopyranoside(GalpαNp), was determined.

Results were shown in Table 2. The Table shows specific activity (units /mg protein) of the β-galactosidase towards above described substrates and relative activity (%).

**[Table 2]**

| Table 2. Specific activity of the β-galactosidase | | |
|---|---|---|
| Substrate | Specific activity | Relative activity |
| | U/mg | % |
| GlcpβNp | 79.2 | 100 |
| GalpβNp | 28.8 | 36.4 |
| ManpβNp | 131.2 | 165.7 |
| XylpβNp | 10.5 | 13.2 |
| FucpβNp | 0 | 0 |
| GalpαNp | 0 | 0 |

Substrate specificity of the β-galactosidase was also determined by using natural substrate as shown in Table 3. The method is shown as follows; each substrate as shown in Table 3 was dissolved in 2ml of 150 mM sodium citrate buffer (pH 5.0) as to give the final concentration equivalent to 17 g/l for carboxymethyl-cellulose, avicel, or laminarin, and to 50mM for the other substrates. After incubating these solutions at 90°C, 5 µl of phosphate buffer (pH 5.0) containing the β-galactosidase is added and the reaction was performed at 90°C for 5 minutes. After ceasing the reaction by cooling with ice, the amount of the liberated glucose was determined by using a glucose measurement kit Glucose C-Test Wako (mfd. by Wako Pure Chemical Industries, Ltd.) One unit of enzyme activity was defined as that amount required to catalyze the hydrolising of 1 µmol of the substrate in a minute. Results were shown in Table 3.

**[Table 3]**

| Table 3. Substrate specificity of the β-galactosidase | |
|---|---|
| Substrate | Relative activity |
| | % |
| Lactose | 100 |
| Cellobiose | 209.3 |
| β-methyl-D-glucoside | 6.5 |
| Salicin | 89.0 |
| Arbutin | 4.5 |
| Sucrose | 0 |
| Maltose | 0 |
| Carboxymethyl-cellulose | 0 |
| Avicel | 0 |
| Laminarin | 0 |

As described above in detail, the present invention provides a gene coding for a hyperthermostable β-galactosidase and a genetic engineering process for producing a hyperthermo-stable β-galactosidase by using this gene. This enzyme has a high thermostability and is useful particularly in food processing at high temperature and saccharide engineering.

The present invention further provides an EcoRI fragment of about 2.2 kbp which codes for the active center of the hyperthermostable β-galactosidase. Incubation of a transformant having a recombinant plasmid containing the above fragment transduced therein gives the hyperthermostable β-galactosidase. This enzyme is not only industrially applicable but also useful in the studies on the thermostabilities of enzymes and so on.

Furthermore, the gene isolated in accordance with the present invention or a gene consisting of a part thereof is useful also as a probe in screening. The use of this gene as a probe makes it easy to clone hyperthermostable β-galactosidase genes hybridizable with this gene from a number of organisms.

The hyperthermostable β-galactosidase obtained via the expression of the hyperthermostable β-galactosidase gene of the present invention can be obtained by incubating a strain belonging to the genus *Pyrococcus* such as *Pyrococcus furiosus* DSM 3638 or *Pyrococcus woesei* DSM 3773 in an appropriate growth medium and purifying the target enzyme from the cells or the culture broth. To incubate a bacterium of the genus *Pyrococcus,* a method usually employed for incubating a hyperthermostable bacterium may be used. Any nutrient which can be utilized by the employed strain may be added to the medium. For example, starch is usable as a carbon source and trypton and peptone are usable as a nitrogen source. As other nutrients, yeast extract and the like can be used. The medium may contain metal salts such as magnesium salts, sodium salts or iron salts as a trace element. It is advantageous to use artificial seawater for the preparation of the medium. The medium is preferably a transparent one free from a solid sulfur element, since such a medium makes it easy to monitor the growth of the cells by measuring the optical density of the culture. The incubation can be effected either stationarily or under stirring. For example, an aeration culture [see Japanese Patent Publication No. 503757/1992] or a dialysis culture [see Appl. Env. Microbiol., S5, 2086 - 2088 (1992)] may be carried out. In general, the incubation temperature is preferably around 95°C. Usually, a considerably large amount of the hyperthermostable β-galactosidase is accumulated in the culture within about 16 hours. It is a matter of course that the incubation conditions should be determined in such a manner as to achieve the maximum yield of the hyperthermo-stable β-galactosidase depending on the selected strain and the composition of the medium.

The hyperthermostable β-galactosidase of the present invention can be harvested by, for example, collecting the cells from the culture broth by centrifuging or filtering and then disrupting the cells. The cell disruption can be effected by, for example, ultrasonic disruption, bead disruption or lytic enzyme treatment. By using these techniques, the hyperthermostable β-galactosidase can be extracted from the cells. The enzyme may be extracted by a method capable of giving the highest extraction effect depending on the selected bacterium and thus a crude enzyme solution is obtained. From the crude enzyme solution thus obtained, the hyperthermostable β-galactosidase can be isolated by combining techniques commonly employed for purifying enzymes, for example, salting out with ammonium sulfate, ion exchange chromatography, hydrophobic chromatography and gel filtration.

For example, a crude enzyme solution prepared from incubated cells of *Pyrococcus furiosus* DSM 3638 is chromatographed with a DEAE Toyopearl M650 ion exchanger (mfd. by Tosoh Corporation) to thereby elute an active fraction. The active fraction thus obtained is poured into an HIC-Cartridge Column (mfd. by Bio-Rad} to thereby elute an active fraction. The active fraction thus eluted is poured into a Superose 12 Column (mfd. by Pharmacia) to thereby elute an active fraction. Thus the hyperthermostable β-galactosidase can be obtained.

### [Brief Description of the Drawings]

### [Fig. 1]

Fig. 1 is a drawing which shows a restriction enzyme map of a plasmid pTGE-101.

### [Fig. 2]

Fig. 2 is a drawing which shows a restriction enzyme map of a plasmid pTGE-301.

### [Fig. 3]

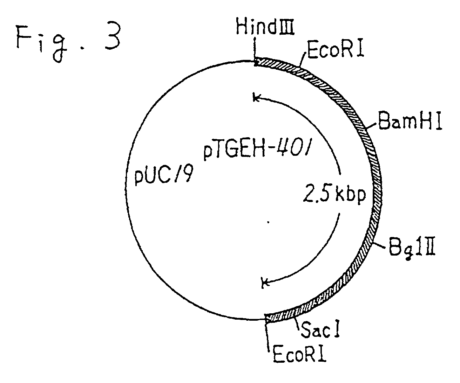

Fig. 3 is a drawing which shows a restriction enzyme map of a plasmid pTGEH-401.

### [Fig. 4]

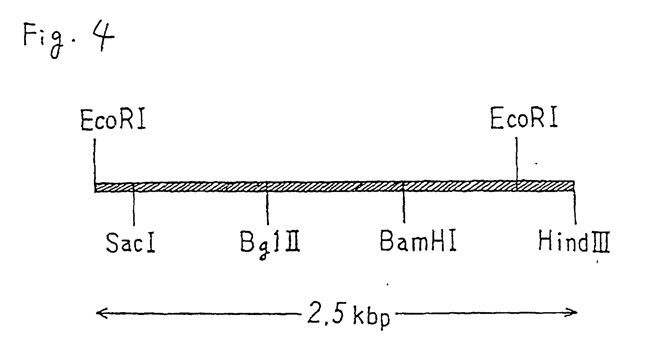

Fig. 4 is a drawing which shows a restriction enzyme map of one example of the hyperthermostable β-galactosidase gene of the present invention.

### [Fig. 5]

Fig. 5 is a graph showing the thermostabilities of the hyperthermostable β-galactosidase of the present invention.

### [Fig. 6]

Fig. 6 is a graph showing the optimum pH of the hyperthermostable β-galactosidase of the present invention.

### [Fig. 7]

Fig. 7 is a graph showing the optimum temperature of the hyperthermostable β-galactosidase of the present invention.

### [Fig. 8]

Fig. 8 is a graph showing the pH stability of the hyperthermostable β-galactosidase of the present invention.

### [Examples]

To further illustrate the present invention in greater detail, and not by way of limitation, the following Examples will be given.

### Example 1

### (1) Preparation of Pyrococcus furiosus genome DNA

*Pyrococcus furiosus* DSM 3638 was incubated in the following manner.

2 1 of a medium comprising 1% of trypton, 0.5% of yeast extract, 1% of soluble starch, 3.5% of Jamarin S Solid (mfd. by Jamarin Laboratory), 0.5% of Jamarin S Liquid (mfd. by Jamarin Laboratory), 0.003% of MgSO₄, 0.001% of NaCl, 0.0001% of FeSO₄·7H₂O, 0.0001% of CoSO₄, 0.0001% of CaCl₂·7H₂O, 0.0001% of ZnSO₄, 0.1 ppm of CuSO₄·5H₂O, 0.1 ppm of KAl(SO₄)₂, 0.1 ppm of H₃BO₃, 0.1 ppm of Na₂MoO₄·2H₂O and 0.25 ppm of NiCl₂·6H₂O was fed into a 2 1 medium bottle and sterilized at 120°C for 20 minutes. After eliminating the dissolved oxygen by blowing nitrogen gas, the medium was inoculated with the above-mentioned strain, which was then stationarily incubated at 95°C for 16 hours. After the completion of the incubation, cells were collected by centrifuging.

Then the collected cells were suspended in 4 ml of a 0.05 M Tris-HCl (pH 8.0) containing 25% of sucrose. To the obtained suspension were added 0.8 ml of lysozyme [5 mg/ml, 0.25 M Tris-HCl (pH 8.0)] and 2 ml of 0.2 M EDTA. After maintaining at 20°C for 1 hour, 24 ml of an SET solution [150 mM NaCl, 1 mM EDTA, 20 mM Tris-HCl (pH 8.0)] was added. Further, 4 ml of 5% SDS and 400 µl of proteinase K (10 mg/ml) were added thereto, followed by a reaction at 37°C for 1 hour. After the completion of the reaction, the reaction mixture was extracted with chloroform/phenol and precipitated from ethanol. Thus approximately 3.2 mg of a genome DNA was prepared.

### (2) Preparation of cosmid protein library

400 µg of the *Pyrococcus furiosus* DSM 3638 genome DNA was partially digested with Sau 3AI in a buffer solution for Sau 3AI [50 mM Tris-HCl (pH 7.5), 10 mM MgCl₂, 1 mM dithio-threitol, 100 mM NaCl] and fractionated according to the size by density gradient centrifugation. One µg of Triple Helix Cosmid Vector was cleaved with Bam HI and mixed with 140 µg of the genome DNA fragments of 35 to 50 kbp which had been obtained by the fractionation as described above. After ligating with the use of a Ligation Kit (mfd. by Takara Shuzo Co., Ltd.), the *Pyrococcus* genome DNA fragments were packaged into λ-phage particles by the *in vitro* packaging method using Gigapack II Gold (mfd. by Stratagene). By using a part of the phage solution thus obtained, *E. coli* DH5αMCR was transformed to thereby give a cosmid library.

From several colonies thus obtained, cosmid DNAs were prepared and it was confirmed that they had an inserted fragment of an appropriate size in common. Next, 500 colonies were suspended in 2 ml of an L-broth medium containing 0.01% of ampicillin and incubated under shaking at 37°C for 16 hours. The culture was centrifuged and cells were collected as a precipitate. These cells were suspended in 20 mM Tris-HCl (pH 8.0) and thermally treated at 100°C for 10 minutes. Subsequently, they were ultrasonicated and further thermally treated at 100°C for 10 minutes. After centrifuging, the supernatant was collected and referred to as a crude enzyme solution. Thus 500 cosmid protein libraries were prepared.

### (3) Selection of cosmid containing β-galactosidase gene

The cosmid protein libraries obtained in the above Example 1-(2) were divided into 50 groups each having 10 cosmid protein libraries. Then the β-galactosidase activity of the crude enzyme solution of each protein library group was determined. Namely, 10 µl of the crude enzyme solution was added to 99.5 µl of a 100 mM phosphate buffer solution (pH 7.3) containing 112 mM of 2-mercaptoethanol and 1 mM of magnesium chloride. After adding 0.5 µl of a dimethyl sulfoxide solution containing 0.4 M of o-nitrophenyl-β-D-galactopyranoside, the mixture was reacted at 95°C for 30 minutes. Then the reaction was ceased by adding 50 µl of 0.1 M sodium carbonate and the absorbance at 410 nm was measured. Thus the amount of the formed o-nitrophenol was determined.

Next, β-galactosidase activities of 60 protein libraries constituting six groups of the cosmid protein libraries showing β-galactosidase activity were further measured. Thus seven cosmid proteins having β-galactosidase activity were specified and seven cosmid DNAs corresponding thereto were specified.

### (4) Preparation of plasmid pTGE-101 and production of thermostable β-galactosidase

Seven cosmid DNAs obtained in the above Example 1-(3) were completely digested with a restriction enzyme EcoRI and electrophoresed on a gel. Thus an EcoRI DNA fragment of about 2.2 kbp which was common to these seven cosmid DNAs was purified. Then a vector pUC19 was cleaved at the EcoRI site and dephosphorylated at the end. Then the above-mentioned EcoRI DNA fragment of about 2.2 kbp was ligated onto this plasmid with the use of a ligation kit. By using the reaction mixture thus obtained, *E. coli* JM109 was transformed. Then a DNA was extracted from the transformant, cleaved with EcoRI and electrophoresed on an agarose gel. Thus the presence of the EcoRI fragment of about 2.2 kbp inserted thereinto was confirmed. The obtained plasmid and the transformant were named respectively pTGE-101 and *Escherichia coli* JM109/pTGE-101.

This EcoRI DNA fragment of about 2.2 kbp was cleaved with several restriction enzymes and analyzed by agarose gel electrophoresis. Fig. 1 shows the results. It is a drawing which shows a restriction enzyme map of the obtained plasmid pTGE-101 having the EcoRI DNA fragment of about 2.2 kbp.

*Escherichia coli* JM109/pTGE-101 was suspended in 5 ml of an L-broth medium containing 0.01% of ampicillin and incubated under shaking at 37°C for 16 hours. The culture was centrifuged and the cells thus collected were suspended in a 50 mM phosphate buffer solution (pH 7.0) containing 1 mM of EDTA and thermally treated at 100°C for 10 minutes. Then the cells were ultrasonicated and further treated at 100°C for 10 minutes. After centrifuging, the supernatant was separated for use as a crude enzyme solution. Then the β-galactosidase activity was determined in accordance with the method as described in the above Example 1-(3) except that 5 µl of this crude enzyme solution was used. As a result, this crude enzyme solution showed no activity. Then another crude enzyme solution was prepared in the same manner as the one described above except that the thermal treatments (100°C, 10 minutes, twice) were omitted. Then the β-galactosidase activity of the crude enzyme solution was determined in accordance with the method of Example 1-(3) except that the reaction temperature was adjusted to 70°C. This crude enzyme solution had an activity of decomposing o-nitrophenyl-β-D-galactopyranoside at pH 7.3 at 70°C. Namely, a gene coding for a thermostable β-galactosidase showing an activity at 70°C had been inserted into pTGE-101.

### (5) Preparation of plasmid pTGH-301

The EcoRI DNA fragment (about 2.2 kbp) obtained in the above Example 1-(4) was labeled with (α-³²P)dCTP (3000 Ci/mmol; mfd. by Amersham) by using a Random Primer Labeling Kit (mfd. by Takara Shuzo Co., Ltd.). By using the labeled DNA fragment thus obtained as a probe, the cosmid DNAs as specified in the above Example 1-(3), which had been completely digested with Hind III and separated by electrophoresis, were analyzed by southern hybridization. Thus a Hind III DNA fragment of about 3.5 kpb hybridized by the above probe was identified and purified. Next, pUC19 was cleaved at the Hind III site and dephosphorylated at the end. The above-mentioned Hind III DNA fragment was ligated onto this plasmid by using a ligation kit and *E. coli* JM109 was transformed thereby. The obtained plasmid was named plasmid pTGH-301, while the transformant was named *Escherichia coli* JM109/pTGH-301. *Escherichia coli* JM109/pTGH-301 was suspended in 5 ml of an L-broth medium containing 0.01% of ampicillin and incubated under shaking at 37°C for 16 hours. The culture was centrifuged and the cells thus collected were suspended in a 50 mM phosphate buffer solution (pH 7.0) containing 1 mM of EDTA and thermally treated at 100°C for 10 minutes. Subsequently, the cells were ultrasonicated and then thermally treated again at 100°C for 10 minutes. After centrifuging, the supernatant was separated for use as a crude enzyme solution. The β-galactosidase activity was determined in accordance with the method of the above Example 1-(3) except that 5 µl of this crude enzyme solution was used. As a result, the activity of a hyperthermostable β-galactosidase which can withstand the thermal treatment at 100°C for 20 minutes was observed in the crude enzyme solution.

The above-mentioned Hind III DNA fragment of about 3.5 kbp was cleaved with several restriction enzymes and analyzed by agarose gel electrophoresis. Fig. 2 shows the results. It is a drawing which shows a restriction enzyme map of the plasmid pTGH-301 containing the Hind III DNA fragment (about 3.5 kbp) obtained above. *E. coli* JM109 transformed by the plasmid pTGH-301 containing the hyper-thermostable β-galactosidase gene was expressed as *Escherichia coli* JM109/pTGH-301 and has been deposited with Fermentation Research Institute of the Agency of Industrial Science and Technology under the accession number FERM P-13348.

### (6) Preparation of plasmid pTGEH-401

The above-mentioned plasmid pTGH-301 was digested with EcoRI and electrophoresed on an agarose gel. Among DNA fragments of about 3.0 kbp, about 2.2 kbp and about 1.0 kbp thus separated, two DNA fragments (of about 3.0 kpb and about 2.2 kbp) were recovered. The DNA fragment of about 3.0 kbp was dephosphorylated with the use of alkaline phosphatase (mfd. by Takara Shuzo Co., Ltd.) and ligated by mixing with the DNA fragment of about 2.2 kbp, followed by transduction into *E. coli* JM109. The hyperthermostable β-galactosidase activities of the colonies thus formed were determined. From colonies showing the activity, a plasmid was prepared and named pTGEH-401. *E. coli* JM109 holding this plasmid was named *Escherichia coli* JM109/pTGEH-401, and deposited at the National Institute of Bioscience and Human-Technology Agency of Industrial Science and Technology, Japan, under the accession number of FERM BP-4468.

Fig. 3 shows a restriction enzyme map of the plasmid pTGEH-401. Fig. 4 shows a restriction enzyme map of the hyperthermostable β-galactosidase gene of about 2.5 kbp, which originates in *Pyrococcus furiosus* and is obtained in accordance with the present invention, inserted into the plasmid pTGEH-401.

### Example 2

### Determination of DNA sequence of the hyperthermostable β-galactosidase gene

The DNA fragment of about 3.5 kbp containing hyperthermostable β-galactosidase gene which have been inserted in the plasmid pTGH-301 was cleaved with several restriction enzymes and subcloned into plasmid vector pUC118. The DNA sequences of each fragment subcloned were determined by the dideoxy method using *Bca*Best™ Dideoxy Sequencing Kit (mfd. by Takara Shuzo. Co.,LTD.). SEQ ID No.2 in SEQUENCE LISTING shows the DNA sequence of the DNA fragment containing the hyperthermostable β-galactosidase gene which have been inserted in the plasmid pTGH-301. SEQ ID No.1 in SEQUENCE LISTING shows the amino acid sequence of the hyperthermostable β-galactosidase which is presumed from the DNA sequence shown in SEQ ID No. 1.

### Example 3

### (1) Production of hyperthermostable β-galactosidase

*Escherichia coli* JM109/pTGH-301 (FERM P-13348) having the plasmid pTGH-301 containing the hyperthermostable β-galactosidase gene of the present invention transduced therein, which was obtained in the above Example 1, was suspended in 5 ml of an L-broth medium containing 0.01% of ampicillin and incubated under shaking at 37°C for 16 hours. Then the culture was suspended in 1.2 1 of a medium of the same conditions and incubated under shaking at 37°C for 16 hours. After centrifuging the culture, the cells thus collected were suspended in a 50 mM phosphate buffer solution (pH 7.0) containing 1 mM of EDTA and thermally treated at 100°C for 10 minutes. Next, the cells were ultrasonicated and thermally treated again at 100°C for 10 minutes. After centrifuging, the supernatant was separated for use as a crude enzyme solution. This crude enzyme solution had 20 U of o-nitrophenyl-β-D-galactopyranoside-decomposing activity per ml of the L-broth medium at pH 7.3 at 95°C. This crude enzyme solution was concentrated with MOLCUT II (cutoff molecular weight: 10,000, mfd. by Millipore Corp.) and gel-filtered through a Superose 12 Column (10 x 300 mm; mfd. by Pharmacia), which had been equilibrated with a 50 mM phosphate buffer solution (pH 7.2) containing 100 mM of NaCl, and an active fraction was collected. The active fraction was concentrated with MOLCUT II (cutoff molecular weight: 10,000; mfd. by Millipore Corp.) and adsorbed by 1 ml of an APTG affinity column (mfd. by MoBi Tec), which had been equilibrated with a 20 mM glycine sodium buffer solution (pH 7.5) containing 10 mM of MgCl₂ and 200 mM of NaCl. After maintaining at room temperature for 1 hour, the column was washed with 12.5 ml of a 20 mM glycine sodium buffer solution (pH 7.5) containing 10 mM of MgCl₂ and 200 mM of NaCl and then eluted with a 100 mM glycine sodium buffer solution (pH 10.0). An active fraction was separated and thus a purified enzyme preparation was obtained. When electro-phoresed on a 7.5% SDS-polyacrylamide gel, this preparation showed a single band.

Similarly, *Escherichia coli* JM109/pTGEH-401 having the plasmid pTGEH-401 containing the hyperthermostable β-galactosidase gene of the present invention transduced therein was incubated in the same manner as the one described above. From the culture, an active fraction of the hyperthermostable β-galactosidase was purified and thus a hyperthermostable β-galactosidase preparation was obtained.

### [Effect of the Invention]

The use of the hyperthermostable β-galactosidase gene of the present invention makes it possible to industrially advantageously produce a hyperthermostable β-galactosidase.

By using the hyperthermostable β-galactosidase gene of the present invention or a part thereof as a probe, hyperthermostable β-galactosidase genes originating in various organisms can be obtained.

## Claims

1. An isolated DNA having:
(a) the DNA sequence of SEQ ID No. 2, or a fragment thereof;
(b) a DNA sequence encoding the amino acid sequence of SEQ ID No. 1 or a fragment thereof;
(c) a DNA sequence encoding the amino acid sequence resulting from deletion, addition, insertion, or substitution, of one or more amino acids in the amino acid sequence of SEQ ID No. 1; or
(d) a DNA sequence capable of hybridizing of any one of (a) to (c) under stringent conditions;
wherein said DNA sequence or fragment thereof encode a polypeptide possessing hyperthermostable β-galactosidase activity of about 100% after having been treated at 90°C for 120 minutes.

2. A process for producing a hyperthermostable β-galactosidase which having the activity of about 100% after having been treated at 90°C for 120 minutes, which comprises:
(a) constructing a plasmid in which an isolated DNA encoding a hyperthermophilic β-galactosidase as claimed in claim 1 is inserted;
(b) transforming an appropriate microorganism with said plasmid; and
(c) cultivating the transformed microorganism and recovering the hyperthermostable β-galactosidase from the culture.

## Patentansprüche

1. Isolierte DNA mit:
(a) der DNA-Sequenz von SEQ ID Nr. 2 oder ein Fragment davon,
(b) einer DNA-Sequenz, die für die Aminosäuresequenz von SEQ ID Nr. 1 oder ein Fragment davon kodiert,
(c) einer DNA-Sequenz, die für die Aminosäuresequenz kodiert, die aus Deletion, Addition, Insertion oder Substitution einer oder mehrerer Aminosäuren in der Aminosäuresequenz von SEQ ID No. 1 resultiert, oder
(d) einer DNA-Sequenz, die in der Lage ist, irgendeine von (a) bis (c) unter stringenten Bedingungen zu hybridisieren,
worin die DNA-Sequenz oder das Fragment davon für ein Polypeptid kodiert, das hyperthermostabile β-Galactosidaseaktivität von ungefähr 100 % besitzt, nachdem es 120 Minuten lang bei 90 °C behandelt wurde.

2. Verfahren zur Herstellung einer hyperthermostabilen β-Galactosidase, die die Aktivität von ungefähr 100 % aufweist, nachdem sie 120 Minuten lang bei 90 °C behandelt wurde, das umfasst:
(a) Konstruieren eines Plasmids, in das eine isolierte DNA durch Insertion eingefügt wird, die für eine hyperthermophile β-Galactosidase kodiert, wie es in Anspruch 1 beansprucht ist,
(b) Transformieren eines geeigneten Mikroorganismus mit dem Plasmid, und
(c) Kultivieren des transformierten Mikroorganismus und Gewinnen der hyperthermostabilen β-Galactosidase aus der Kultur.

## Revendications

1. ADN isolé ayant :
(a) la séquence d'ADN de la SEQ ID N° 2, ou un de ses fragments ;
(b) une séquence d'ADN codant pour la séquence d'aminoacides de la SEQ ID N° 1 ou un de ses fragments ;
(c) une séquence d'ADN codant pour la séquence d'aminoacides résultant de la délétion de l'addition, de l'insertion ou de la substitution d'un ou plusieurs aminoacides dans la séquence d'aminoacides de la SEQ ID N° 1 ; ou
(d) une séquence d'ADN apte à l'hybridation avec l'une quelconque des séquences (a) à (c) dans des conditions drastiques ;
dans lequel ladite séquence d'ADN ou son fragment code pour un polypeptide possédant une activité de β-galactosidase hyperthermostable d'environ 100 % après traitement à 90°C pendant 120 minutes.

2. Procédé pour la production d'une β-galactosidase hyperthermostable ayant une activité d'environ 100 % après traitement à 90°C pendant 120 minutes, qui comprend :
(a) la construction d'un plasmide dans lequel un ADN isolé codant pour une β-galactosidase hyperthermophile suivant la revendication 1 est insérée ;
(b) la transformation d'un micro-organisme approprié avec ledit plasmide ; et
(c) la culture du micro-organisme transformé et la séparation de la β-galactosidase hyperthermostable de la culture.
